# EUROPEAN PATENT APPLICATION

(11) **EP 4 818 147 A1**
(43) Date of publication of application: **07.10.2026**
(21) Application number: 24897111.1
(22) Date of filing: 11.10.2024
(51) Int. Cl.: C07C 41/38, C07C 43/23

(54) **PRODUCTION METHOD FOR GENERATING 3,5-DIHYDROXY-4-METHOXYBENZYL ALCOHOL FROM OYSTER MEAT**

(30) Priority: 28.11.2023 JP 2023200390
(71) Applicant: Watanabe Oyster Laboratory Co., Ltd., Hachioji-Shi Tokyo 192-0154 (JP)
(72) Inventor: WATANABE, Mitsugu, Hachioji-shi, Tokyo 192-0154 (JP)
(74) Representative: Plougmann Vingtoft a/s
(86) International application number: PCT/JP2024/036387
(87) International publication number: WO 2025/115421

(57) **Abstract**

[Problem]

The present invention provides pressurizing and extracting frozen raw oyster meat at the initial stage to allow further extraction of DHMBA from raw oyster meat, and allow the extraction of high-concentration DHMBA in a short period and in large quantities.

[Solution]

The present invention includes charging raw oysters into a container holding an extraction solvent, sealing and pressurizing the container, concentrating the pressurized extraction solvent to generate a highly concentrated solution including 3,5-dihydroxy-4-methoxybenzyl alcohol, and generating 3,5-dihydroxy-4-methoxybenzyl alcohol from the concentrated solution.

## Description

### TECHNICAL FIELD

The present invention relates to a method for producing 3,5-dihydroxy-4-methoxybenzyl alcohol from oyster meat.

### BACKGROUND ART

Conventionally, the method for producing 3,5-dihydroxy-4-methoxybenzyl alcohol (hereinafter referred to as DHMBA) from raw oysters is a method for heating raw oysters at one atmosphere for one hour, extracting components from the raw oysters and transferring them to an extract, then removing the boiled oysters, and reheating or pressurizing the extract to generate DHMBA.

### CITATION LIST

### PATENT DOCUMENT

Patent Document 1: Japanese Patent Laid-Open No. 2012-153852

### DISCLOSURE OF THE INVENTION

### PROBLEMS TO BE SOLVED BY THE INVENTION

However, the inventor of the present invention had concerns that, for example, when raw oysters are heated and extracted at one atmosphere for one hour, then the boiled oysters are removed, and the extract is reheated or pressurized to generate DHMBA, the nutrients in the raw oysters are not sufficiently extracted. The inventor believed that more nutrients remained in the boiled oysters that were removed.

That is, instead of heating raw oysters, performing the extraction process, removing the raw oysters, and then reheating and pressurizing the remaining extract to generate DHMBA from the extract, as in the conventional method, the inventor thought that if the raw oysters themselves, for example frozen raw oysters, are placed in a solvent, heated, and directly pressurized, more nutrients could be extracted from the oysters. In particular, it is presumed that a large amount of DHMBA can be extracted.

As described above, placing and heating the raw oysters themselves in a solvent and then directly performing pressurized extraction makes it possible to efficiently obtain a large amount of oyster extract including nutrients such as DHMBA in a shorter period.

To further explain the process leading to the present invention, the inventor of the present invention, at the time of founding the company, did not own a pressure vessel, and thus frozen raw oyster meat was heated at one atmosphere, then the boiled oyster meat was removed, the remaining oyster meat extract was concentrated and allowed to be cooled naturally to room temperature, then the concentrated oyster meat solution was mixed with, for example, 99.9% ethanol and allowed to settle, and separated into a supernatant and a precipitate. Thereafter, he succeeded in creating the novel antioxidant DHMBA from the supernatant.

Then, the inventor of the present invention focused on developing a technique to increase the DHMBA concentration in the supernatant obtained by heating at one atmosphere. The inventor then found that the DHMBA concentration was able to be increased by heating and pressurizing the extract obtained by heating oyster meat at one atmosphere, and obtained various patents.

However, in recent years, it has been suspected that heating at one atmosphere alone may not be sufficient to extract DHMBA from frozen raw oyster meat.

The inventor of the present invention hypothesized that it would be possible to extract even more DHMBA from the raw oyster meat by pressurizing and extracting frozen raw oyster meat at the initial stage, potentially allowing the extraction of high-concentration DHMBA in a short period and in a large amount.

The inventor of the present invention already owns a laboratory-scale pressure vessel and a plant-scale pressure vessel. Therefore, it was possible to smoothly perform pressurized heating extraction experiments in which frozen raw oyster meat is subjected to pressurized extraction at the initial stage.

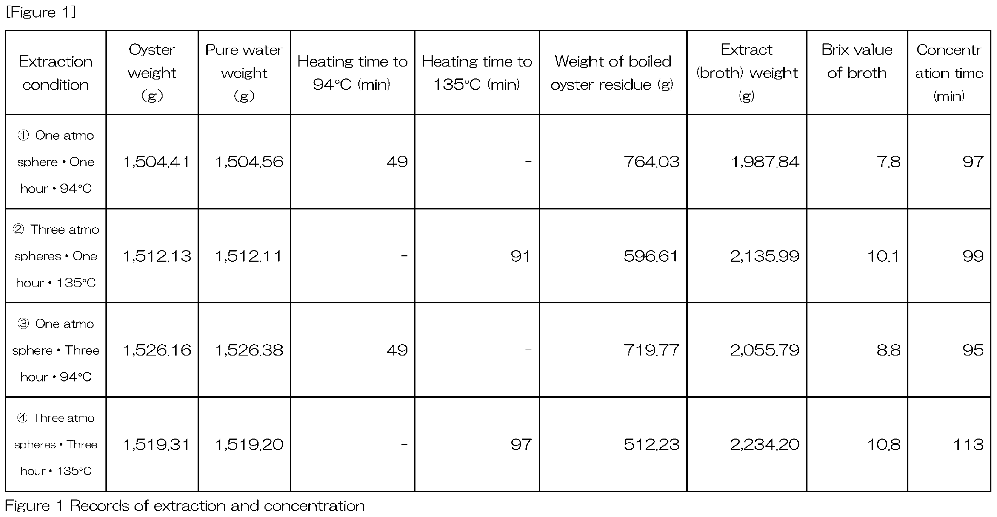

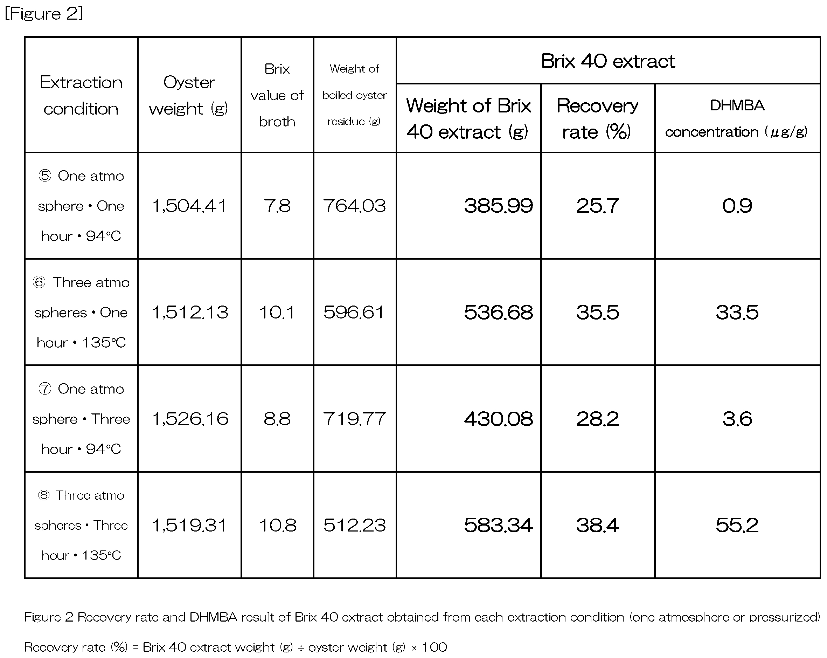

As shown in Figures 1 and 2, pressurized heating extraction of the frozen raw oyster meat at the initial stage makes it possible to produce a high-concentration DHMBA-containing extract from the frozen raw oyster meat, further, in a large amount. Further, it was confirmed that a high-concentration DHMBA-containing extract was able to be produced in a short period.

### SOLUTIONS TO THE PROBLEMS

The present invention includes:
charging raw oysters into a container holding an extraction solvent, sealing and pressurizing the container, concentrating the pressurized extraction solvent to generate a highly concentrated solution including 3,5-dihydroxy-4-methoxybenzyl alcohol, and generating 3,5-dihydroxy-4-methoxybenzyl alcohol from the concentrated solution;
charging raw oysters into a container holding an extraction solvent, sealing and pressurizing the container, concentrating the pressurized extraction solvent to generate a concentrated solution including 3,5-dihydroxy-4-methoxybenzyl alcohol with a high recovery rate, and generating 3,5-dihydroxy-4-methoxybenzyl alcohol from the concentrated solution;
charging frozen raw oysters into a container holding an extraction solvent approximately equal in weight to the oysters, sealing and pressurizing the container, concentrating the pressurized extraction solvent to generate a highly concentrated solution including 3,5-dihydroxy-4-methoxybenzyl alcohol, and generating 3,5-dihydroxy-4-methoxybenzyl alcohol from the concentrated solution; or
charging frozen raw oysters into a container holding an extraction solvent approximately equal in weight to the oysters, sealing and pressurizing the container, concentrating the pressurized extraction solvent to generate a concentrated solution including 3,5-dihydroxy-4-methoxybenzyl alcohol with a high recovery rate, and generating 3,5-dihydroxy-4-methoxybenzyl alcohol from the concentrated solution, wherein
the pressurization is to increase the pressure to one atmosphere or more;
the extraction solvent is water; or
the container is a pressure vessel.

### EFFECTS OF THE INVENTION

With the method for producing 3,5-dihydroxy-4-methoxybenzyl alcohol from oyster meat according to the present invention, more nutrients can be extracted from the oysters by heating raw oysters themselves, for example frozen raw oysters, in a solvent and then directly pressurizing them. In particular, it is presumed that a large amount of DHMBA can be extracted.

Placing and heating the raw oysters themselves in a solvent, and then directly performing pressurized extraction exhibits such an excellent effect that a large amount of oyster extract including nutrients such as DHMBA can be more efficiently obtained in a shorter period.

### BRIEF DESCRIPTION OF THE DRAWINGS

Figure 1 shows a table for explaining the measurement conditions of the present invention.
Figure 2 shows a table for explaining the measurement results of the present invention.

### DESCRIPTION OF PREFERRED EMBODIMENTS

The following experiments for generating 3,5-dihydroxy-4-methoxybenzyl alcohol (DHMBA) actually using frozen raw oysters were performed for the present invention, and the experimental examples are explained with reference to the figures.

### (Experimental materials)

Frozen oyster blocks from Hiroshima were used as experimental materials. That is, the raw oysters used in each experiment performed under a set of conditions were all thawed from the same frozen oyster block from Hiroshima.

In addition, the amount of frozen oyster block from Hiroshima used in each experiment performed under a set of conditions was set to 1.5 kg.

Pure water was used as the solvent for heating the raw oysters, and in the experiments performed under each set of conditions, the amount of pure water used under each condition was 1.5 kg. Therefore, the ratio between oysters and water was 1:1.

In addition, the pressure vessel used was a pressure vessel (OAMVP-005) manufactured by KAJIWARA INC.

### (Extraction conditions)

First, the conditions for the extraction experiments performed under each of the conditions described above are as follows.
(1) oyster:water = 1:1, extracted at 94°C for one hour (1 atm, 1 hour)
(2) oyster:water = 1:1, extracted at 135°C for one hour (3 atm, 1 hour)
(3) oyster:water = 1:1, extracted at 94°C for three hours (1 atm, 3 hours)
(4) oyster:water = 1:1, extracted at 135°C for three hours (3 atm, 3 hours)

After extraction, the mixture was concentrated under reduced pressure to obtain a Brix of 40.

Herein, Brix represents the concentration of solid components dissolved in a solvent, and a concentrate of oyster broth with a Brix of 40% is called a Brix 40 concentrate. However, the measurement is not limited to a Brix 40 concentrate in which the oyster broth is concentrated to a Brix of 40%, and may be performed using a concentrate concentrated to any concentration (%).

### (Extraction Method)

6.0 kg of oysters (1.5 kg × 4 conditions) used in the present experiment were thawed from a 10 kg frozen oyster block. After thawing, the weight of the oysters used in each of the conditions described above was measured and recorded. Thereafter, an amount of pure water equal to the weight of the oysters was prepared.

As described above, a pressure vessel (OAMVP-005) manufactured by KAJIWARA INC. was used for extraction from the oysters. An equal amount of 1.5 kg of each of the oysters and pure water were charged into the pressure vessel.

After charging, the lid of the vessel was closed, and under extraction conditions (1) and (3), heating was performed until the temperature inside the vessel reached 94°C. When 94°C was reached, the pressure was one atmosphere, and under this one atmosphere, heating extraction was performed at 94°C for one hour in (1) and at 94°C for three hours in (3).

The pressure vessel was equipped with a pressure gauge, and one atmosphere state was maintained when 94°C was reached. Herein, as long as one atmosphere state is maintained, the heating temperature is not limited to 94°C.

In the extraction conditions of (2) and (4), the lid and pressure valve of the vessel were closed, and pressurization was performed until the temperature inside the vessel reached 135°C. When 135°C was reached, the pressure gauge indicated that three atmospheres were maintained. Then, under the three atmospheres, pressurized extraction was performed at 135°C for one hour in (2) and at 135°C for three hours in (4).

Note that the three-atmosphere state was maintained when 135°C was reached, and the heating temperature is not limited to 135°C, as long as the three-atmosphere state is maintained.

After extraction was completed, the Brix value and weight of the extract (broth) obtained under each condition, as well as the weight of the boiled oyster residue after extraction, were measured.

The broth under each condition was concentrated using a rotary evaporator until the Brix value of the broth reached 40. At this time, the temperature of the water bath of the rotary evaporator was set to 95°C. After concentration was completed, the weight of the Brix 40 extract obtained from the broth under each condition was measured. The measured weight of each Brix 40 extract was divided by the weight of the oysters used for extraction to calculate the recovery rate (yield) of the weight of the Brix 40 extract from the weight of the oysters.

In addition, the DHMBA concentration in each Brix40 extract was quantified using a liquid chromatogram-tandem mass spectrometer (LC-MS/MS: Prominence highpressure gradient HPLC, triple quadrupole mass spectrometer LCMS-8040 system, Shimadzu Corporation).

The weights of oysters and pure water used under each extraction condition, the required heating and pressurizing time, and the values related to concentration are shown in Table 1 of Figure 1.

### (Measurement details)

The items measured this time are as follows:
1. Recovery rate of Brix 40 extract weight from raw oyster weight This is because a high recovery rate of Brix 40 extract weight is expected to indicate a high recovery rate of DHMBA.
2. DHMBA concentration in Brix 40 extract

This is because a high DHMBA concentration in Brix 40 extract is expected to indicate a large amount of DHMBA.

The above measurement results are shown in Figure 2.

### (Conclusion)

As shown in the above results, the weight of Brix 40 extract obtained was larger with three atmospheres of pressurized extraction than with one atmosphere of extraction. This indicates that three atmospheres of pressurized extraction were able to extract more nutrients from the oysters compared to one atmosphere of extraction, and the DHMBA concentration was also higher with three atmospheres of pressurized extraction.

In the previous extraction method for which the inventor of the present invention has obtained a patent, for example, after extracting nutrients from oysters at one atmosphere state, the oysters are removed and only the extract is heated or pressurized to generate DHMBA.

In this extraction method, the oysters are removed before pressurization, and the extraction time for the oysters is short, for example, one hour, and thus it is thought that a large amount of important nutrients, including DHMBA, remain in the oysters.

As shown in Figure 2, the novel pressurized extraction method was confirmed to increase the amount of DHMBA extracted and generated from raw oysters in the initial stage, that is, by directly pressurizing the raw oysters, compared to the previous patented method.

Further, the method for placing the oysters directly in pure water, heating, and pressurizing for extraction can perform the extraction process in a significantly shorter period than the method described in the existing patent held by the inventor of the present invention.

As described above, the extraction method of the present invention, which involves pressurizing raw oysters directly from the initial stage of extraction from raw oysters, can be said to be a superior method for producing extract from raw oysters and generating DHMBA compared to the conventional extraction method.

That is, the present invention, which involves pressurizing and extracting raw oysters directly, can provide a large amount of DHMBA more efficiently and in a shorter period than the existing patent.

## Claims

1. A method for producing 3,5-dihydroxy-4-methoxybenzyl alcohol from oyster meat, comprising
charging raw oysters into a container holding an extraction solvent, sealing and pressurizing the container, concentrating the pressurized extraction solvent to generate a highly concentrated solution including 3,5-dihydroxy-4-methoxybenzyl alcohol, and generating 3,5-dihydroxy-4-methoxybenzyl alcohol from the concentrated solution.

2. A method for producing 3,5-dihydroxy-4-methoxybenzyl alcohol from oyster meat, comprising
charging raw oysters into a container holding an extraction solvent, sealing and pressurizing the container, concentrating the pressurized extraction solvent to generate a concentrated solution including 3,5-dihydroxy-4-methoxybenzyl alcohol with a high recovery rate, and generating 3,5-dihydroxy-4-methoxybenzyl alcohol from the concentrated solution.

3. A method for producing 3,5-dihydroxy-4-methoxybenzyl alcohol from oyster meat, comprising
charging frozen raw oysters into a container holding an extraction solvent approximately equal in weight to the oysters, sealing and pressurizing the container, concentrating the pressurized extraction solvent to generate a highly concentrated solution including 3,5-dihydroxy-4-methoxybenzyl alcohol, and generating 3,5-dihydroxy-4-methoxybenzyl alcohol from the concentrated solution.

4. A method for producing 3,5-dihydroxy-4-methoxybenzyl alcohol from oyster meat, comprising
charging frozen raw oysters into a container holding an extraction solvent approximately equal in weight to the oysters, sealing and pressurizing the container, concentrating the pressurized extraction solvent to generate a concentrated solution including 3,5-dihydroxy-4-methoxybenzyl alcohol with a high recovery rate, and generating 3,5-dihydroxy-4-methoxybenzyl alcohol from the concentrated solution.

5. The method for producing 3,5-dihydroxy-4-methoxybenzyl alcohol from oyster meat according to claim 1 or 2,
wherein the pressurization is to increase a pressure to one atmosphere or more.

6. The method for producing 3,5-dihydroxy-4-methoxybenzyl alcohol from oyster meat according to claim 1 or 2,
wherein the extraction solvent is water.

7. The method for producing 3,5-dihydroxy-4-methoxybenzyl alcohol from oyster meat according to claim 1 or 2,
wherein the container is a pressure vessel.
